# EUROPEAN PATENT APPLICATION

(11) **EP 3 118 276 A1**
(43) Date of publication of application: **18.01.2017**
(21) Application number: 15760926.4
(22) Date of filing: 09.03.2015
(51) Int. Cl.: C09K 3/00, A61K 9/14, B01D 9/02, B01J 2/10, B01J 13/00, B82Y 5/00, C07C 13/62, C07C 15/20, C07C 15/38, C07C 22/08, C07C 63/44, C07C 69/616, C07C 233/80, C07D 235/20, C07D 309/10, C07D 401/12, C07F 7/18, C07F 9/53, C07J 9/00

(54) **WATER-DISPERSIBLE AMORPHOUS PARTICLES AND METHOD FOR PREPARING SAME**

(30) Priority: 10.03.2014 US 201461950282 P
(71) Applicant: The University of Tokyo, Tokyo 113-8654 (JP); TOWA PHARMACEUTICAL CO., LTD., Kadoma-shi, Osaka 5718580 (JP)
(72) Inventor: NAKAMURA, Eiichi, Tokyo 113-8654 (JP); HARANO, Koji, Tokyo 113-8654 (JP); INAKOSHI, Naoto, Kadoma-shi Osaka 571-8580 (JP); LIU, Chao, Kadoma-shi Osaka 571-8580 (JP)
(74) Representative: Krauns, Christian
(86) International application number: PCT/JP2015/056879
(87) International publication number: WO 2015/137289

(57) **Abstract**

The present invention pertains to spherical water-dispersible amorphous particles having a particle diameter of 10-990 nm and a PDI of 0.01-0.5, wherein the amorphous particles contain an organic compound having a molecular weight of 50-1500, and a method for preparing the same.

## Description

### Field of the Invention

The present invention relates to water-dispersible amorphous particles and a method for preparing the same.

### Description of the Related Art

Submicrometer-sized particles have wide applicability in the pharmaceutical manufacturing field, printing technology field, organic electronic device field, etc.

Submicrometer-sized inorganic particles, semiconductor particles and polymer particles can be easily prepared, and have been widely researched (Non-Patent Documents 1 to 3).

### Citation List

### Non-Patent Documents

Non-Patent Document 1: Xu, L. G. et al., Chem Soc. Rev. 2013, 42, 3114
Non-Patent Document 2: Sakaino, H. et al., J. Am. Chem. Soc. 2012, 134, 15684
Non-Patent Document 3: Kuehne, A. J. C. et al., Nat. Commun. 2012, 3, 1088

### Summary of the Invention

### Technical Problem

No example, however, has been reported with respect to preparation of submicrometer-sized spherical amorphous particles comprising a low molecular organic compound as a constituent component.

Accordingly, an object of the present invention is to provide submicrometer-sized spherical amorphous particles comprising a low molecular organic compound, and a method for preparing the same.

### Solution to Problem

In view of the above problem, the present inventors have made intensive studies, and as a result, have found that either an organic solution, in which an organic compound is dissolved, or water can be introduced to the other and the organic solution and water can be mixed to thereby prepare submicrometer-sized spherical amorphous particles comprising the organic compound, with high reproducibility, thereby leading to the present invention.

The present invention includes the following aspects.
[1] Spherical water-dispersible amorphous particles having a particle size of 10 nm to 990 nm and having a PDI of 0.01 to 0.5, comprising an organic compound having a molecular weight of 50 to 1500.
[2] The amorphous particles according to [1], wherein the organic compound has a logP of 2.0 or higher.
[3] The amorphous particles according to [1] or [2], wherein the organic compound has a solubility in water of 0.3 mg/ mL or less at 25°C.
[4] The amorphous particles according to any one of [1] to [3], wherein the organic compound is selected from the group consisting of formoterol, lansoprazole, cholesterol, simvastatin and telmisartan, and pharmaceutically acceptable salts thereof and solvates thereof.
[5] The amorphous particles according to any one of [1] to [4], wherein the amorphous particles are a dried product.
[6] A method for preparing the amorphous particles according to any one of [1] to [5], comprising the following steps of:
   (1) dissolving an organic compound in a water-miscible organic solvent to prepare an organic solution; and
   (2) introducing either the organic solution or water to the other, and mixing the organic solution and water to prepare a dispersion of the amorphous particles.
[7] The method according to [6], wherein the concentration of the organic compound in the organic solution prepared in the step (1) is 0.1 µM to 1000 mM.
[8] The method according to [6] or [7], wherein the mixing of the organic solution and water in the step (2) is performed by injecting the organic solution into water while stirring water.
[9] The method according to [8], wherein the injection speed of the organic solution to X mL of water is 0.01X mL/min to 10X mL/min.
[10] The method according to [6] or [7], wherein the mixing of the organic solution and water in the step (2) is performed by injecting water into the organic solution while stirring the organic solution.
[11] The method according to [10], wherein the injection speed of water to Y mL of the organic solution is 0.01Y mL/min to 10Y mL/min.
[12] The method according to [6] or [7], wherein the mixing of the organic solution and water in the step (2) is performed by contacting the organic solution and water by flowing them.
[13] The method according to [12], wherein the mixing of the organic solution and water in the step (2) is performed by use of a microreactor.
[14] The method according to any one of [6] to [13], further comprising stirring the dispersion of the amorphous particles prepared in the step (2) at 1 to 60°C to thereby increase the particle size of the amorphous particles.
[15] The method according to any one of [6] to [14], further comprising evaporating the organic solvent in the dispersion of the amorphous particles prepared in the step of (2) to prepare a water dispersion of the amorphous particles.
[16] The method according to any one of [6] to [15], further comprising preparing a dried product of the amorphous particles from the dispersion of the amorphous particles.

### Advantageous Effects of the Invention

According to the method of the present invention, submicrometer-sized spherical amorphous particles comprising a low molecular organic compound can be prepared simply with high reproducibility. The resulting particle has wide applicability in the pharmaceutical manufacturing field, printing field, organic electronic device field, etc.

In particular, in the pharmaceutical field, the amorphous particles of the present invention can exert the following excellent effects.

### (1) Effect of solubility enhancement

An active component of a pharmaceutical product has recently tended to have a complex structure for the purpose of enhancement in activity, to thereby have a higher lipophilicity, and as a result, has often caused the problem of solubility in water. The particle of the present invention is an amorphous particle, and therefore can have higher solubility than a crystalline particle of the same compound.

### (2) Effect due to submicrometer size

The amorphous particles of the present invention have a small particle size, and therefore can contribute to improve solubility of the active component. In addition, the amorphous particles of the present invention are effective for use in an inhalation formulation. As a specific example, a dry powder inhalation formulation is considered as an application, which is a dosage form for use as a therapeutic agent for bronchial asthma. In order to exhibit the medicinal effect of the formulation, the active component may be preferably small to the extent that it can reach a deep part of the lung. Pulverization by a hammer mill or a jet mill is generally used as the method for micronizing the active component, but the pulverization method has a limitation in micronizing. The amorphous particles of the present invention can serve as an ideal inhalation formulation.

### (3) Effect due to spherical particle

While the particle shape of the active component is desirably a uniform spherical shape in terms of operability in a manufacturing process, such a particle has been difficult to obtain. In addition, a technique is generally used in formulating, such as a bitterness masking technique as a response to bitterness of the active component, and an enteric coating technique for dissolution and absorption not in the stomach but in the intestine. The shape of a particle to be coated in the above techniques is neither needle-shaped nor plate-shaped, but is desirably a uniform spherical shape as much as possible. A uniform spherical particle can have a smaller specific area and can be coated with a smaller amount of a masking substrate than needle-shaped and plate-shaped particles, and can allow both quality and productivity of a formulation itself to be enhanced. Furthermore, the active component can be spherical also in terms of countermeasures to tabletting problems resulting in a smaller specific area for suppression of adherence to the surface of a beetle in tabletting, resulting in suppression of the risk of tabletting troubles. The amorphous particles of the present invention are spherical, and therefore can be expected to exert the above effects.

### Brief Description of the Drawings

[Figure 1] Figure 1 depicts a scanning electron microscope (SEM) image of amorphous particles spin-coated on indium tin oxide (ITO) from its water dispersion obtained in Example 1.
[Figure 2] Figure 2 depicts a SEM image of amorphous particles spin-coated on indium tin oxide (ITO) from its water dispersion obtained in Example 2.
[Figure 3] Figure 3 illustrates the change in particle size of amorphous particles according to the change in concentration of a THF solution.
[Figure 4] Figure 4 illustrates the change in particle size of amorphous particles, which depends on the change in injection speed of injecting THF solution of PhH into water.
[Figure 5] Figure 5 illustrates the change in particle size of amorphous particles in a dispersion over time.
[Figure 6] Figure 6 illustrates the analysis results by a dynamic light scattering (DLS) method of a water dispersion of amorphous particles obtained in Example 9.
[Figure 7] Figure 7 depicts a SEM image of a freeze-dried product of the amorphous particles obtained in Example 9.
[Figure 8] Figure 8 illustrates the powder X-ray diffraction result of the freeze-dried product of the amorphous particles obtained in Example 9.
[Figure 9] Figure 9 depicts a SEM image of amorphous particles spin-coated on indium tin oxide (ITO) from water dispersion of the particles obtained in Example 10.
[Figure 10] Figure 10 depicts a SEM image of a freeze-dried product of the amorphous particles obtained in Example 10.
[Figure 11] Figure 11 illustrates the powder X-ray diffraction result of the freeze-dried product of the amorphous particles obtained in Example 10.
[Figure 12] Figure 12 illustrates the analysis results by a DLS method of a dispersion of amorphous particles obtained in Example 11.
[Figure 13] Figure 13 depicts a SEM image of a freeze-dried product of the amorphous particles obtained in Example 11.
[Figure 14] Figure 14 illustrates the analysis results by a DLS method of a dispersion of an amorphous particles obtained in Example 12.
[Figure 15] Figure 15 depicts a SEM image of a freeze-dried product of the amorphous particles obtained in Example 12.
[Figure 16] Figure 16 illustrates the powder X-ray diffraction result of the freeze-dried product of the amorphous particles obtained in Example 12.
[Figure 17] Figure 17 illustrates the analysis results by a DLS method of a dispersion of an amorphous particles obtained in Example 13.
[Figure 18] Figure 18 depicts a SEM image of a freeze-dried product of the amorphous particles obtained in Example 13.
[Figure 19] Figure 19 illustrates the powder X-ray diffraction result of the freeze-dried product of the amorphous particles obtained in Example 13.
[Figure 20] Figure 20 is a schematic view of a reaction in Example 1.
[Figure 21] Figure 21 is a schematic view of a reaction apparatus in Example 11. A Y-shaped microreactor provided with two liquid supply passages and a confluence passage where the two liquid supply passages meet is illustrated.

### Description of Embodiments

One aspect of the present invention relates to spherical water-dispersible amorphous particles having a particle size of 10 nm to 990 nm and having a PDI of 0.01 to 0.5, comprising an organic compound having a molecular weight of 50 to 1500. Hereinafter, the amorphous particles are also referred to as "the amorphous particles of the present invention".

The "submicrometer" herein means 1 nm or more and less than 1 µm. Particles having a submicrometer-sized particle size are herein also referred to as "submicrometer particles (Submicron particles)" or "SMPs". Hereinafter, the amorphous particles of the present invention are also referred to as "SMPs of the present invention".

The "amorphous particle" herein means a particle that is not in a crystalline state. Whether a particle is amorphous or not can be determined, for example, by whether a diffraction peak is represented in powder X-ray diffraction or not, by scanning electron microscope observation, or by selected-area electron diffraction with a transmission electron microscope, without particular limitation.

The "particle size" herein means an average particle size, and is defined as a particle size (Z-average) measured by use of a dynamic light scattering (Dynamic Light Scattering: DLS) method. The particle size thus measured is also referred to as "hydrodynamic diameter".

The amorphous particles of the present invention have a particle size of 10 nm to 990 nm, preferably 30 nm to 500 nm.

The "polydispersity index (Polydispersity Index: PDI)" herein is an index for evaluating the width of a particle size distribution, and is in the range from 0 to 1. A value of 0 represents an ideal suspension without any size distribution. A distribution having a PDI value of 0.1 or less is referred to as "monodispersity", and on the other hand, a dispersion having a value between 0.1 and 0.3 is considered to have a narrow size distribution. A dispersion having a PDI of more than 0.5 is considered to be polydisperse. The polydispersity index is calculated from the value obtained by use of a dynamic light scattering (Dynamic Light Scattering: DLS) method.

The amorphous particles of the present invention have a polydispersity index (Polydispersity Index: PDI) of 0.01 to 0.5, preferably 0.01 to 0.2.

The amorphous particles of the present invention comprise an organic compound having a molecular weight of 50 to 1500, preferably having a molecular weight of 200 to 800, substantially consist of the organic compound, or consist of the organic compound. When the amorphous particles consist of the organic compound, for example, impurities that can be incorporated during preparation of the amorphous particles of the present invention may be comprised in the amorphous particle.

The organic compound that is a constituent component of the amorphous particles of the present invention may be in any of a free form (i.e., a composite with other compound is not formed), a salt thereof (preferably a pharmaceutically acceptable salt thereof, when a free compound is a biologically functional substance or medicine) and a solvate thereof, or may be a mixture thereof.

The "salt" herein is not particularly limited, and examples thereof include salts with inorganic acids such as sulfuric acid, hydrochloric acid, hydrobromic acid, phosphoric acid and nitric acid, salts with organic acids such as acetic acid, oxalic acid, lactic acid, tartaric acid, fumaric acid, maleic acid, citric acid, benzenesulfonic acid, methanesulfonic acid, p-toluenesulfonic acid, benzoic acid, camphorsulfonic acid, ethanesulfonic acid, glucoheptonic acid, gluconic acid, glutamic acid, glycolic acid, malic acid, malonic acid, mandelic acid, galactaric acid and naphthalene-2-sulfonic acid, salts with one or more metal ions of a lithium ion, a sodium ion, a potassium ion, a calcium ion, a magnesium ion, a zinc ion, an aluminum ion, etc., and salts with amines such as ammonia, arginine, lysine, piperazine, choline, diethylamine, 4-phenylcyclohexylamine, 2-aminoethanol and benzathine. The "pharmaceutically acceptable salt" herein means a salt of a free compound, the salt being pharmaceutically acceptable and having a desired pharmacological activity. The pharmaceutically acceptable salt may be any of the above salts as long as it satisfies such conditions.

The "solvate" herein means a molecular composite of a free compound or a salt compound and one or more solvent molecules. The solvate is not particularly limited, and examples thereof include hydrates and alcohol solvates (for example, a methanol solvate, an ethanol solvate, a propanol solvate and an isopropanol solvate).

The amorphous particles of the present invention have a spherical shape. The "spherical shape" herein means that the general average sphericity is 0.80 or more, preferably 0.85 or more, more preferably 0.90 or more, particularly preferably 0.95 or more. The "sphericity" is one of the indices that represent the degree of spherical shape of a particle. The sphericity of a true sphere is defined to be 1.0, and a sphericity closer to 1.0 means that the shape of a particle is more similar to a true sphere. A specific measurement method is as follows. A two-dimensional image of a particle is taken and an analysis treatment of the image is performed to calculate the length L of the circumference and the area S of the particle, and thereafter ϕ represented by ϕ = 4πΣS/L² is calculated as the sphericity. For example, a particle image can be taken by use of an electron microscope, and thereafter an image analysis treatment with image analysis software (for example, WinROOF (manufactured by Mitani Corporation)) can be performed to thereby calculate the sphericity. The "average sphericity" herein is obtained as follows: the sphericity is determined for 100 particles randomly selected from a sample, and the average is defined as the average sphericity.

The amorphous particles of the present invention are water-dispersible. The "water-dispersible" herein means that a compound forms an emulsion, a micro-emulsion or a suspension in water at a normal temperature.

The partition coefficient "logP" herein represents, as a common logarithm, the concentration ratio of an objective substance in a 1-octanol layer to that in an aqueous layer when the objective substance is added to a mixed solution of 1-octanol and water and then the system reaches equilibrium, and is a general parameter representing the hydrophobicity of a substance. A compound having a higher logP value exhibits a higher hydrophobicity. On the other hand, a compound having a lower logP value exhibits a higher hydrophilicity. The logP of the organic compound herein is a measured value described in the literature, and those of fullerene derivatives and polycyclic aromatic hydrocarbons are calculated values calculated by using ACD/ChemSketch version 14.01 (Advanced Chemistry Development, Inc.).

The organic compound for use in the amorphous particles of the present invention is not particularly limited, and it is preferably an organic compound having a certain level of hydrophobicity. For example, the logP is preferably 2.0 or more, more preferably 4.0 or more.

The organic compound for use in the amorphous particles of the present invention preferably has solubility in water of 0.3 mg/ mL or less at 25°C.

The organic compound for use in the present invention is not particularly limited, and examples thereof include the following compounds.
(1) Fullerene derivatives represented below, such as C8H, DPPF, PC₆₁BM, PC₇₁BM, PCBNB, PhH, SIMEF2 and F1H. 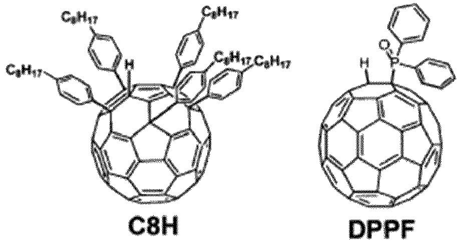 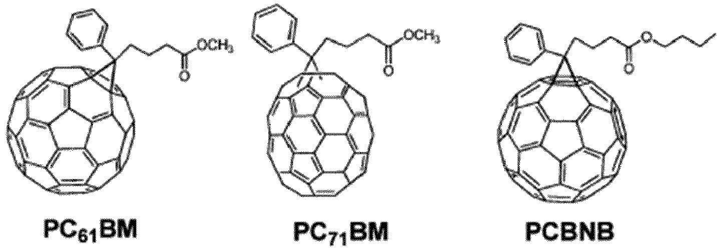 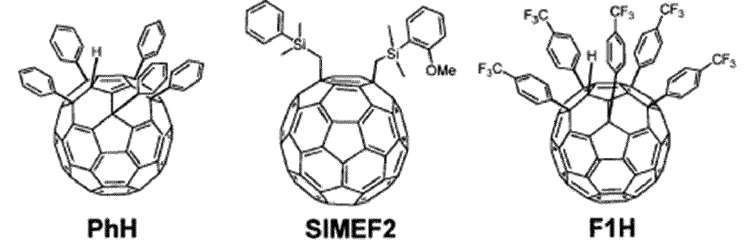
(2) Polycyclic aromatic hydrocarbons such as pentacene, pyrene and pyrenecarboxylic acid (PyCOOH). 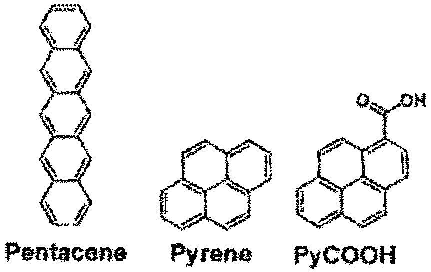
(3) Compounds such as formoterol, lansoprazole, cholesterol, simvastatin and telmisartan, pharmaceutically acceptable salts thereof (for example, formoterol fumarate), or solvates thereof (for example, hydrates or alcohol solvates).

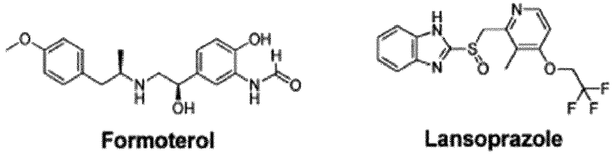

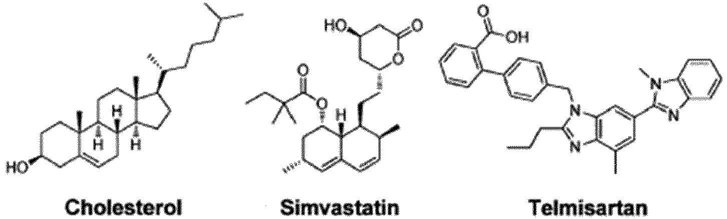

The amorphous particles of the present invention may be present as a dispersion in a mixed solvent of water and an organic solvent, or may be present as a water dispersion. Alternatively, it may be present as a dried product (for example, freeze-dried product).

One aspect of the present invention relates to a method for preparing the amorphous particles of the present invention, comprising the following steps of:
(1) dissolving an organic compound in a water-miscible organic solvent to prepare an organic solution; and
(2) introducing either the organic solution or water to the other and mixing the organic solution and water to prepare a dispersion of the amorphous particles. Hereinafter, the preparation method is also referred to as "the method of the present invention".

In the first step of the method of the present invention, the organic compound is dissolved in the water-miscible organic solvent to prepare the organic solution of the organic compound. The organic solvent is not particularly limited as long as it is water-miscible, and for example, is a lower alcohol (a linear or branched, mono-ol, diol or triol having 1 to 6 carbon atoms, for example, methanol, ethanol, propanol, isopropanol, butanol, isobutanol, tert-butanol, ethylene glycol, propylene glycol, glycerin, diethylene glycol or diethylene glycol monoethyl ether), tetrahydrofuran, acetone, N,N-dimethylformamide, dimethylsulfoxide, or a mixed solvent thereof. The organic solvent is preferably a solvent having a boiling point lower than that of water, more preferably methanol and tetrahydrofuran. The concentration of the organic compound in the organic solution is not particularly limited, and it is preferably 0.1 µM to 100 mM, more preferably 0.1 mM to 100 mM, particularly preferably 1 mM to 10 mM. The organic compound for use in the present step may be a solvate (for example, hydrate or alcohol solvate) of the organic compound as a component of the amorphous particles prepared by the method of the present invention.

In the second step of the method of the present invention, the organic solution of the organic compound, and water are mixed to prepare the dispersion of the amorphous particle. The mixing step is performed by introducing either the organic solution or water to the other.

The "introduction" in the mixing step described above is the initial stage of the mixing step in which mixing of the organic solution and water is finally performed. The introduction of either the organic solution or water to the inside of the other herein is particularly referred to as "injection". The "introduction" also encompasses contact of either the organic solution or water with the other at the interface between them in order to mix them. The "introduction" does not encompass dropping of either the organic solution or water onto the liquid surface of the other.

When either the organic solution or water is introduced to the other, such introduction is preferably performed while the other flowing. Alternatively, such introduction is also preferably performed while both of them flowing. The "flowing" may be, for example, flowing by stirring of the organic solution and/or water in a reaction vessel, or flowing by feeding of the organic solution and/or water into a reaction tube.

The pH of water for use in the method of the present invention may be acidic, neutral or alkaline, as long as stability of the organic compound as a substance is not impaired.

The volume ratio of the organic solution to water for use in the method of the present invention (organic solution: water) is not particularly limited, and it is preferably 1:1 to 1:100, more preferably 1:2 to 1:10.

The mixing temperature in the mixing step can be arbitrarily determined within the range from the freezing point to the boiling point of the solvent to be used, and it is preferably 1 to 40°C, preferably 15 to 30°C.

One embodiment of the mixing step is performed by injecting the organic solution into water while stirring water. The injecting method is not particularly limited, and, for example, it is performed by use of a syringe, with the tip of the needle being placed in water. The injection speed of the organic solution is preferably kept constant. More preferably, the organic solution is injected at a constant injection speed at a stretch. The injection speed of the organic solution to X mL of water is preferably 0.01X to 10X mL/min, more preferably 0.1X to 5X mL/min. The range of X is not particularly limited, and it is preferably 1.0 ≤ X ≤ 4.0 × 10⁶. The stirring speed is preferably 100 rpm to 1000 rpm, more preferably 300 rpm to 500 rpm.

Another embodiment of the mixing step is performed by injecting water into the organic solution while stirring the organic solution. The injection method is not particularly limited, and, for example, the organic solution is injected by use of a syringe, with the tip of the needle being placed in the organic solution. The injection speed of water is preferably kept constant. More preferably, water is injected at a constant injection speed at one time. The injection speed of water to Y mL of the organic solution is preferably 0.01Y to 10Y mL/min, more preferably 0.1Y to 5Y mL/min. The range of Y is not particularly limited, and it is preferably 1.0 ≤ Y ≤ 1.0 × 10⁶. The stirring speed is preferably 100 rpm to 1000 rpm, more preferably 300 rpm to 500 rpm.

Another embodiment of the mixing step is performed by contacting the organic solution and water at the interface between them while flowing them. The organic solution and water preferably form a laminar flow. The laminar flow means the one that moves in streamlines and that has Reynolds number of 2300 or less. The present embodiment is preferably performed by use of a microreactor.

The microreactor (micro flow reactor) is defined as an apparatus comprising a microchannel generally having an equivalent diameter of several mm or less, preferably less than 1000 µm, and the reaction proceeds in the microchannel. The equivalent diameter means a diameter which the area of the circle with is equal to the cross-sectional area of the channel. In the microreactor in which the microchannel is a micro scale, both of the dimension and the flow rate are small, and the flow of the fluid in the microreactor becomes a laminar flow state. Two fluids flow in the channel in the laminar flow state, in which the reaction undergoes between the molecules that are diffusing spontaneously.

In addition to a microreactor in which mixing occurs by the diffusion of a molecule as its spontaneous behavior, equipped with no particular mixing device, there is another type of microreactor that can use a micro flow reaction element or a static micromixer to perform the reaction in the steady state. The static micromixer is an apparatus typified by, for example, a mixer having a narrow channel for mixing, e.g., described in WO 96/30113, or a mixing device (mixer) described in ""Microreactors", vol. 3, edited by W. Ehrfeld, V. Hessel and H. Lowe, published by Wiley-VCH".

The microreactor for use in the present invention can be selected from existing microreactors, commercially available microreactors, and newly designed prototype microreactors for the intended reaction. Examples of the commercially available microreactors include a microreactor having an interdigital channel structure, a single mixer and a caterpillar mixer manufactured by Institut fur Mikrotechnik Mainz (IMM) GmbH; a micro-glass reactor manufactured by Mikroglas Chemtech GmbH; Cytos manufactured by CPC Systems GmbH; KeyChem mixer manufactured by YMC Co., Ltd.; Model YM-1 mixer and Model YM-2 mixer manufactured by Yamatake Corp.; Mixing Tee and Tee (T-shaped connector, Y-shaped connector) manufactured by Shimadzu GLC Ltd.; IMT chip reactor manufactured by Institute of Microchemical Technology Co., Ltd.; Micro High Mixer developed by Toray Engineering Co., Ltd.; and a central collision mixer (K-M type), and any of them can be used in the present invention.

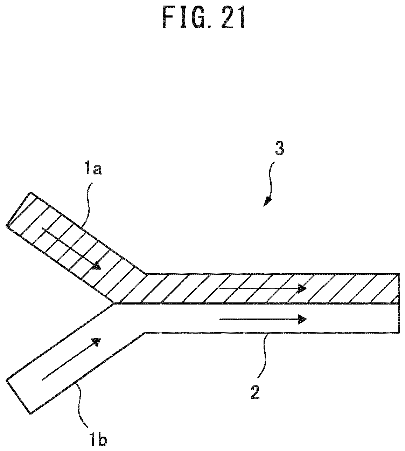

One example where the mixing step is performed by use of a Y-shaped microreactor is illustrated in Figure 21. Liquids to be mixed are allowed to flow through liquid inlet channels 1a and 1b. Laminar flows of both the liquids are contacted at the interface between the liquids in a confluence passage 2 to be combined, and finally mixed by diffusion of molecules.

When the mixing step is performed by use of a microreactor, the feed speeds of the organic solution and water can be arbitrarily determined as long as these do not exceed performances of an experiment apparatus to be used. The feed speeds of the organic solution and water may be the same or different. The feed speeds of the organic solution and water can be the same or different, and preferably 0.01 to 50 mL/min.

In the method of the present invention, a prepared dispersion of the amorphous particles in organic solvent/water can be stirred at 1 to 60°C to increase the particle size of the amorphous particles along with a lapse of time. Such an increase in the particle size is considered to be resulted from the following: the remaining organic solvent serves as a solvent to induce monomer dissociation from the particle, thereby allowing Ostwald ripening to progress. An optional organic solvent may be added. The volume of the organic solvent included in the dispersion is preferably 10% to 40% based on the volume of the entire dispersion. The increase in the particle size of the amorphous particles can be terminated by evaporation of the organic solvent. Thus, the particle size of the amorphous particles can be easily controlled.

The organic solvent in the dispersion of the amorphous particles prepared by the method of the present invention may be evaporated and removed to prepare the water dispersion of the amorphous particle. Evaporation of the organic solvent are performed, for example, by use of a rotary evaporator under reduced pressure.

A dried product of the amorphous particles can be if necessary prepared from the dispersion of the amorphous particles prepared by the method of the present invention. The method for such preparation is not particularly limited as long as the amorphous particles can be kept amorphous and spherical, and a method commonly used, such as evaporation of the solvent at normal pressure or under reduced pressure, drying under reduced pressure, freeze-drying, a spin-coating method or a drop casting method can be used. Preferably, freeze-drying is adopted. The dried product of the amorphous particles prepared by the method of the present invention is one aspect of the present invention, and the drying method is as described above, for example.

The amorphous particles prepared by the method of the present invention is very stable, and for example, can be kept dispersed and spherical in water for at least several months.

In addition, the method of the present invention can be applied to thereby prepare amorphous organic particles even from an organic compound with high crystallinity (for example, pentacene).

### Examples

The present invention is described with reference to Examples and Comparative Example below in more detail, but the scope of the present invention is not, needless to say, limited to these Examples.

Various reagents used in Examples were commercially available products unless especially noted. PhH, C8H, DPPF, SIMEF2 and F1H as fullerene derivatives were prepared according to documents (M. Sawamura, et al. J. Am. Chem. Soc., 1996, 118, 12850-12851; H. Isobe, et al. Org. Lett., 2005, 7, 5633-5635; T. Homma, et al. J. Am. Chem. Soc., 2011, 133, 6364-6370; and H. Tanaka, et al. Adv. Mater. 2012, 24, 3521-3525).

Measurement by a DLS method was performed by use of a laser light scattering apparatus (Zetasizer Nano ZS manufactured by Malvern Instruments Ltd.). A scanning electron microscope (SEM) image was obtained by use of Magellan 400L manufactured by FEI Company. Powder X-ray diffraction was performed by use of SmartLab manufactured by Rigaku Corporation. FDU-1200 manufactured by EYELA was used for a freeze-dryer.

### [Example 1]

### Production of water-dispersible amorphous particles comprising PhH (a pentaphenyl adduct of [60] fullerene (Ph₅C₆₀H) ) as constituent component

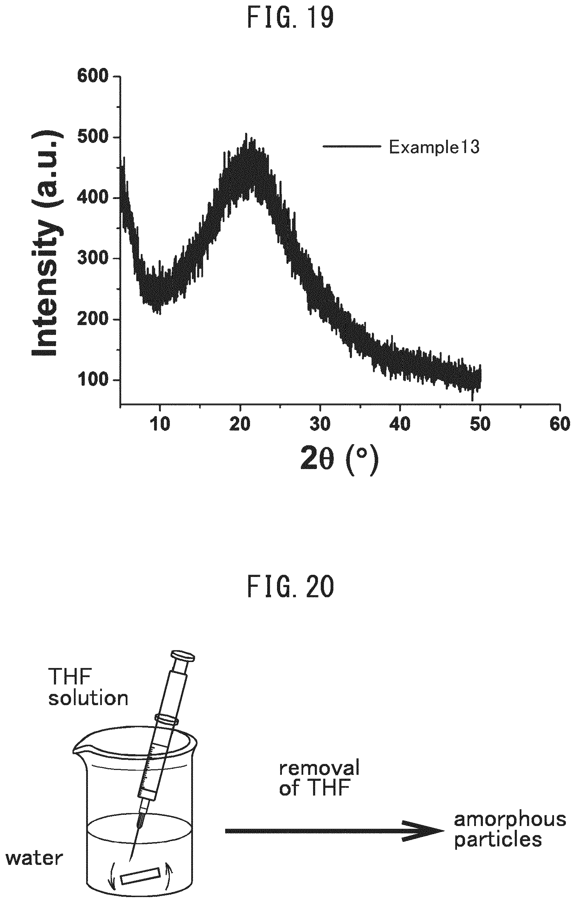

28 mg of PhH was dissolved in 50 mL of THF at room temperature to prepare a 500 µM solution of PhH. 5 mL of ultrapure water and a magnetic stirrer were placed in a flat bottom glass vial, and stirred at a rate of 400 rpm. 5 mL of the THF solution was taken in a gastight syringe, and the syringe was mounted to a syringe pump, and disposed so that the tip of a syringe needle was at the center of the solution. While stirring being continued, 1.2 mL of the THF solution of PhH was injected for 1 minute. After stirring was stopped, the resulting solution was transferred to an eggplant-shaped flask, and THF was rapidly distilled off by a rotary evaporator (the decompression degree: 70 Torr) to thereby provide a water dispersion of amorphous particles. A schematic view of the Example is illustrated in Figure 20.

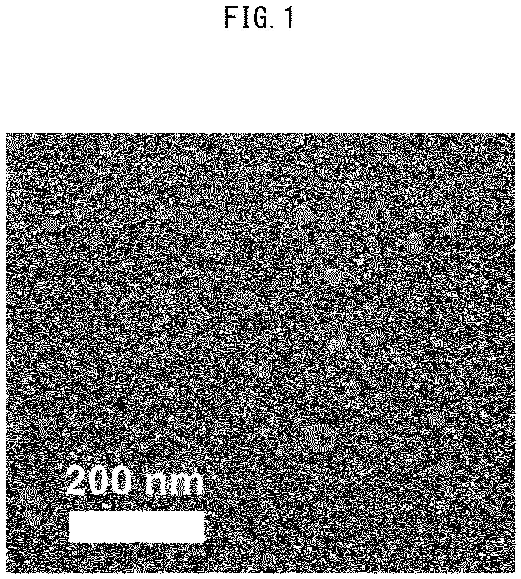

Water was added to the resulting water dispersion so that the constant amount was 5 mL, and the particle size (hydrodynamic diameter) measured by a laser light scattering apparatus was 44 nm. The PDI was 0.09. The dispersion was spin-coated on Indium tin oxide (ITO), and observation by SEM provided an image of spherical amorphous particles illustrated in Figure 1. The dispersion was transferred to an eggplant-shaped flask and thereafter frozen, and freeze-drying (the decompression degree: 10 Pa) was performed in a freeze-drying machine to thereby provide 0.67 mg of a red solid. The resulting solid was confirmed by powder X-ray diffraction to exhibit amorphous property.

### [Example 2]

The same manner as in Example 1 was performed except that the operation for injecting the THF solution of PhH to ultrapure water in Example 1 was changed to an operation for injecting ultrapure water to the THF solution of PhH. In other words, 1.2 mL of the THF solution of PhH and a magnetic stirrer were placed in a flat bottom glass vial, and stirred at a rate of 400 rpm. 10 mL of ultrapure water was taken in a gastight syringe, and the syringe was mounted to a syringe pump, and disposed so that the tip of a syringe needle was at the center of the solution. While stirring being continued, 5 mL of ultrapure water was injected over 1 minute.

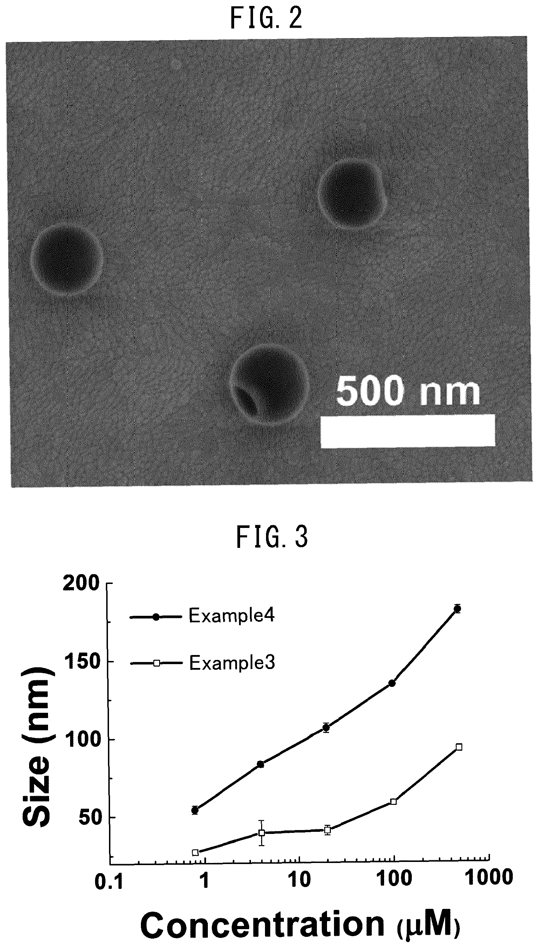

The particle size of the amorphous particles (hydrodynamic diameter) was measured in the same manner as in Example 1, and was determined to be 96 nm. The PDI was 0.04. In addition, the resulting water dispersion was spin-coated onto indium tin oxide (ITO) and observation by SEM provided an image of spherical amorphous particles illustrated in Figure 2. In addition, the water dispersion was freeze-dried to thereby provide a solid. The resulting solid was confirmed by powder X-ray diffraction to exhibit amorphous property.

### [Example 3]

The same manner as in Example 1 was performed except that the concentration of the THF solution of PhH in Example 1 was changed to 100 µM, 20 µM, 4 µM or 0.8 µM. The change in the particle size of the amorphous particles according to the change in the concentration of the THF solution is illustrated in Figure 3. It was confirmed that as the concentration of the THF solution was increased, the particle size of the amorphous particles was increased.

### [Example 4]

The same manner as in Example 2 was performed except that the concentration of the THF solution of PhH in Example 2 was changed to 100 µM, 20 µM, 4 µM or 0.8 µM. The change in the particle size of the amorphous particles according to the change in the concentration of the THF solution is illustrated in Figure 3. It was confirmed that as the concentration of the THF solution was increased, the particle size of the amorphous particles was increased.

### [Example 5]

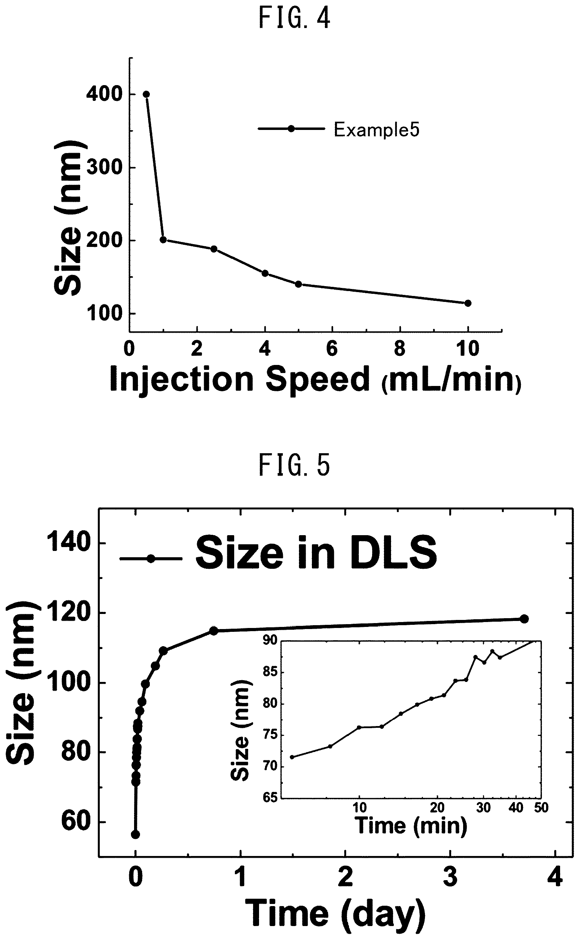

The same manner as in Example 1 was performed except that the injection speed of the THF solution in Example 1 was changed to 0.5 mL/min, 1 mL/min, 2.5 mL/min, 4 mL/min, 5 mL/min, or 10 mL/min. The change in the particle size of the amorphous particles according to the change in the injection speed is illustrated in Figure 4. It was confirmed that as the injection speed of the THF solution was increased, the particle size of the amorphous particles was decreased.

### [Example 6]

The same manner as in Example 1 was performed except that a compound recited in Table 1 was used instead of PhH in Example 1 and the concentration of the THF solution of the compound was 100 µM. In any case, water-dispersible spherical amorphous particles could be favorably prepared. The particle size and the PDI value of the resulting amorphous particles are represented in Table 1.

**Table 1**

| Compound name | Molecular weight | logP | Particle size (nm) | PDI |
|---|---|---|---|---|
| C8H | 1671.3 | 40.66 | 41 | 0.08 |
| DPPF | 922.8 | 12.95 | 50 | 0.13 |
| PC61BM | 910.9 | 14.23 | 88 | 0.09 |
| PC71BM | 1031.0 | 16.32 | 77 | 0.07 |
| PCBNB | 953.0 | 15.82 | 44 | 0.08 |
| SIMEF2 | 1049.3 | 20.28 | 48 | 0.07 |
| F1H | 1452.2 | 19.67 | 30 | 0.13 |
| Simvastatin | 418.6 | 4.72 | 126 | 0.38 |
| Cholesterol | 386.6 | 9.61 | 228 | 0.27 |

### [Example 7]

The same manner as in Example 2 was performed except that a compound recited in Table 2 was used instead of PhH in Example 2 and the concentration of the THF solution of the compound was 100 µM. In any case, water-dispersible amorphous particles could be favorably prepared. The particle size and the PDI value of the resulting amorphous particles are represented Table 2.

**Table 2**

| Compound name | Molecular weight | logP | Particle size (nm) | PDI |
|---|---|---|---|---|
| C8H | 1671.3 | 40.66 | 126 | 0.03 |
| DPPF | 922.8 | 12.95 | 142 | 0.16 |
| PC₆₁BM | 910.9 | 14.23 | 170 | 0.42 |
| PC₇₁BM | 1031.0 | 16.32 | 106 | 0.07 |
| PCBNB | 953.0 | 15.82 | 95 | 0.2 |
| SIMEF2 | 1049.3 | 20.28 | 90 | 0.1 |
| F1H | 1452.2 | 19.67 | 130 | 0.02 |
| Pentacene | 278.4 | 7.14 | 116 | 0.02 |
| Pyrene | 202.3 | 5.14 | 120 | 0.13 |
| Pyrenecarboxylic acid | 246.3 | 4.85 | 76 | 0.14 |
| Simvastatin | 418.6 | 4.72 | 93 | 0.44 |
| Cholesterol | 386.6 | 9.61 | 165 | 0.02 |
| Telmisartan | 514.6 | 6.48 | 10 | 0.34 |

Interestingly, even when pentacene which is known to be an easily-crystallizable compound was used, the method of the present invention could be applied to thereby provide an amorphous particle.

### [Example 8]

### Control of particle size of water-dispersible amorphous particles by addition of THF

Into the water dispersion of the amorphous particles including PhH as a constituent component, prepared in Example 1, THF was injected whose volume rate was 30%. After injection of THF, the particle size of the amorphous particles was increased along with a lapse of time (Figure 5).

### [Example 9]

### Preparation of amorphous particles comprising formoterol fumarate as constituent component

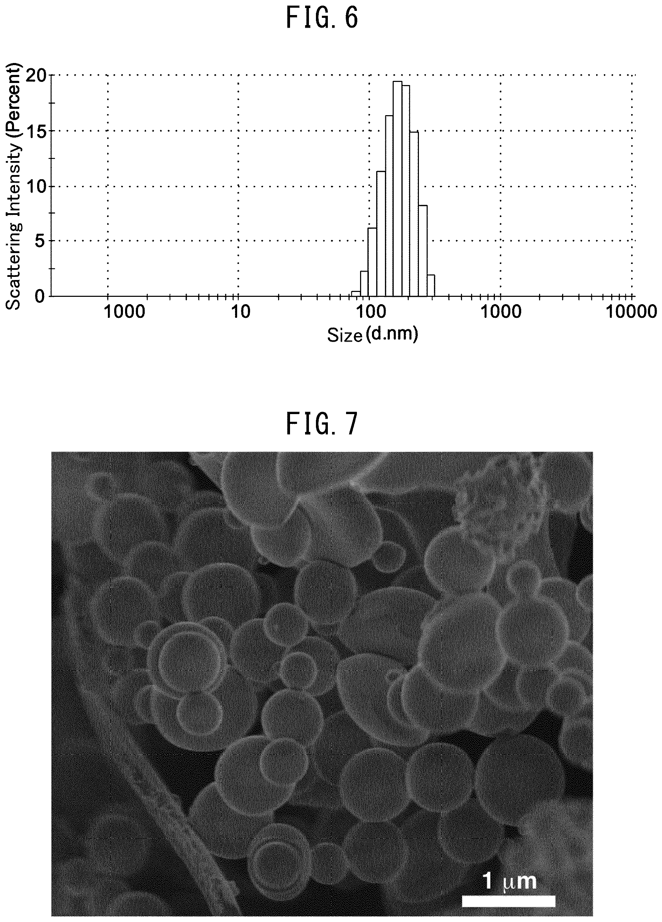

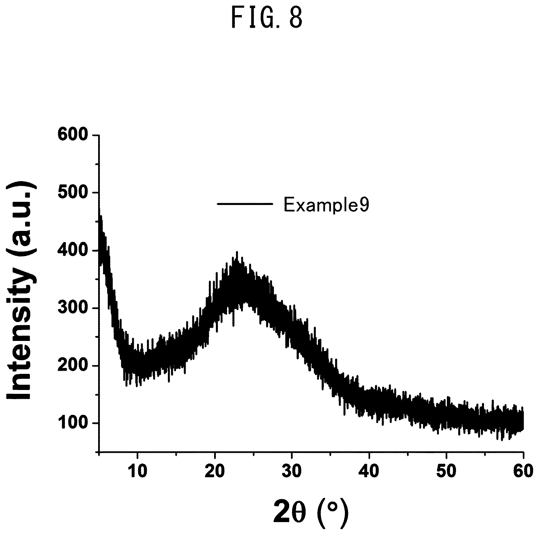

Formoterol fumarate hydrate (molecular weight: 840.9, logP = 2.0, solubility in water: 0.17 mg/ mL at 25°C) was dissolved in THF to prepare a 1 mM solution, and 10 mL of the solution was placed in a 50-mL glass bottle. While the solution in the glass bottle was vigorously stirred (400 rpm) by a magnetic stirrer, 40 mL of purified water was continuously injected into the glass bottle at room temperature over 1 minute. Stirring was stopped, a mixed liquid was concentrated by a rotary evaporator under reduced pressure to provide a liquid, and the liquid was analyzed by a DLS method to provide a particle size distribution illustrated in Figure 6. The concentrated liquid was freeze-dried to provide amorphous particles as a white powder, comprising formoterol fumarate as a constituent component. A sample in which a part of the resulting powder was secured to an aluminum pin stub by use of a double-faced carbon tape was prepared, and observed by SEM to provide a spherical particle image illustrated in Figure 7. Furthermore, the powder was subjected to powder X-ray diffraction measurement, and was found to be amorphous by a diffraction pattern illustrated in Figure 8.

### [Example 10]

### Preparation of amorphous particles comprising lansoprazole as constituent component

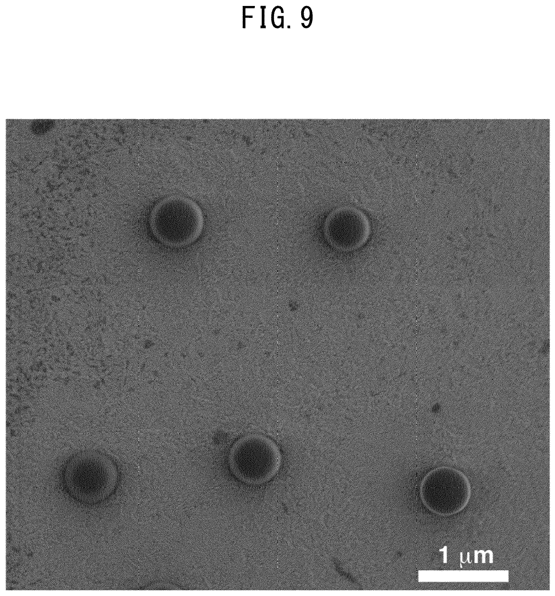

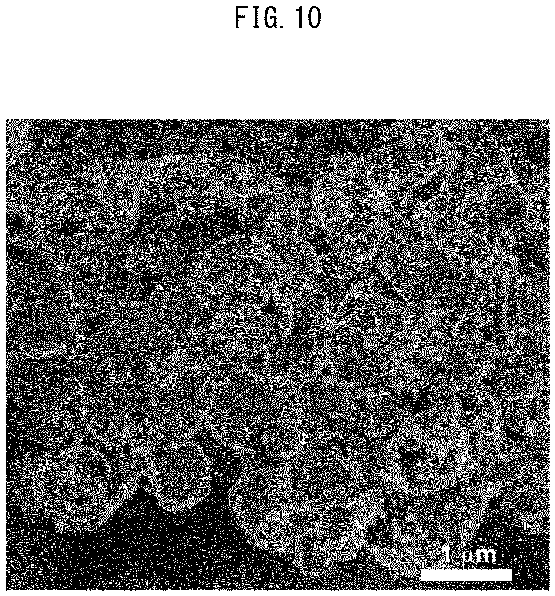

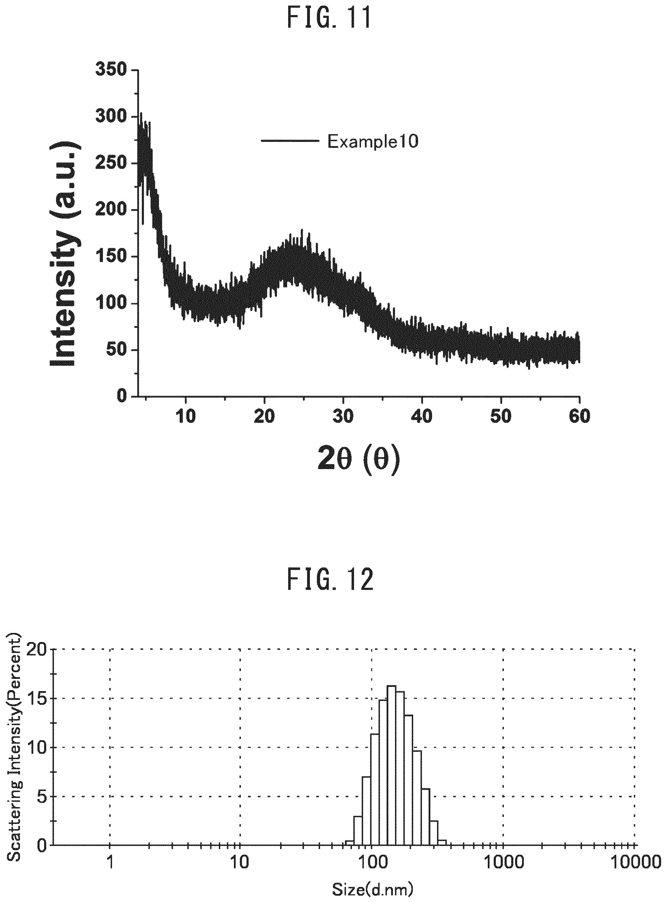

Lansoprazole (molecular weight: 369.3, logP = 2.58, solubility in water: less than 0.1 mg/ mL at 25°C) was dissolved in THF to prepare a 1 mM solution, and 1.25 mL of the solution was placed in a glass bottle having a volume of 10 mL. While the solution in the glass bottle was vigorously stirred (400 rpm) by a magnetic stirrer, 10 mL of purified water was continuously injected into the glass bottle at room temperature over 1 minute. Stirring was stopped, a mixed liquid was concentrated by a rotary evaporator under reduced pressure to provide a liquid, and the liquid was analyzed by a DLS method. In addition, concentrated liquid was spin-coated onto indium tin oxide (ITO), and observation by SEM provided an image of spherical amorphous particles illustrated in Figure 9. The liquid was freeze-dried to provide amorphous particles as a white powder, comprising lansoprazole as a constituent component. An SEM sample was prepared on double-faced carbon tape, fixed to an aluminum pin stub and then observed by SEM to provide a spherical particle image illustrated in Figure 10. Furthermore, the powder was subjected to powder X-ray diffraction measurement, and was found to be amorphous by a diffraction pattern illustrated in Figure 11.

The analysis results by a DLS method of the amorphous particles powders obtained in Example 9 and Example 10 are represented in Table 3.

**Table 3**

| Example | particle size (nm) | PDI |
|---|---|---|
| Example 9 | 162 | 0.015 |
| Example 10 | 167 | 0.13 |

### [Example 11]

### Preparation of amorphous particles comprising formoterol fumarate as constituent component by use of microreactor

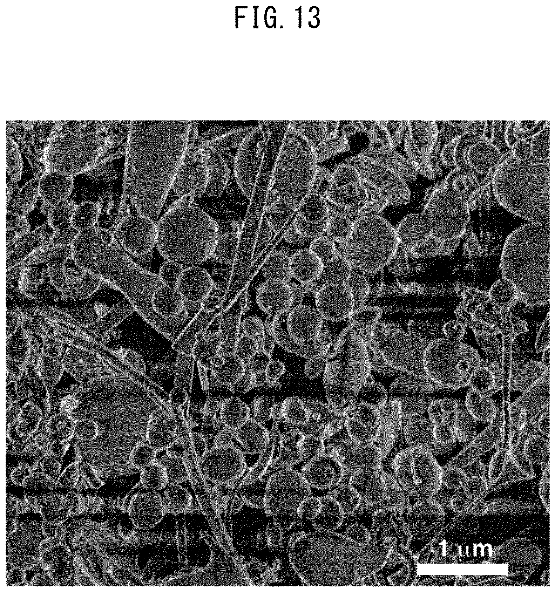

Formoterol fumarate hydrate was dissolved in THF to prepare a 0.5 mM solution (liquid A). On the other hand, purified water was prepared and defined as liquid B. Two syringe pumps were used to allow liquid A and liquid B to flow in a stainless micromixer (Y-shaped) (KeyChem mixer Model: KC-M-Y-SUS manufactured by YMC Co., Ltd.) having a channel size of 0.5 mm (width) × 0.1 mm (depth) and a mixing portion volume of 1.4 µL at a flow rate of 0.01 mL/min and a flow rate of 0.04 mL/min, respectively, at room temperature for 1 minute so that liquid A and liquid B were mixed. A schematic view of a reaction apparatus in the present Example is illustrated in Figure 21. Liquid A was allowed to flow through a liquid supply passage 1a, liquid B was allowed to flow through a liquid supply passage 1b, and laminar flows of both the liquids were contacted at the interface between them, combined in a confluence passage 2, and finally mixed by molecular diffusion. The mixed liquid was analyzed by a DLS method to provide particle size distribution (particle size: 141 nm, PDI: 0.124) illustrated in Figure 12. Subsequently, concentration under reduced pressure was performed by a rotary evaporator to provide a concentrated liquid, and the concentrated liquid was freeze-dried to provide amorphous particles as a white powder, comprising formoterol fumarate as a constituent component. An SEM sample was prepared on double-faced carbon tape, fixed to an aluminum pin stub and then observed by SEM to provide a spherical particle image illustrated in Figure 13.

### [Example 12]

### Preparation of amorphous particles comprising formoterol fumarate as constituent component

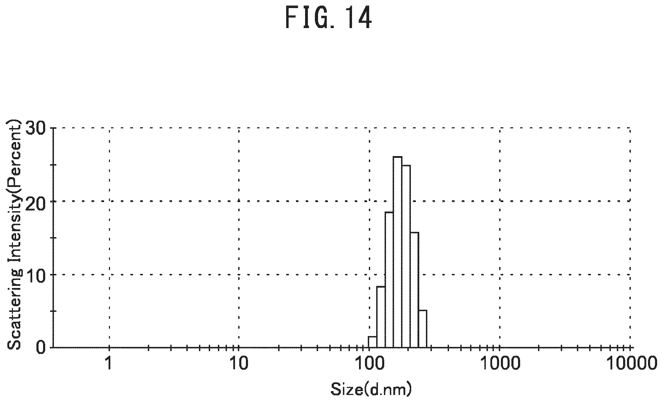

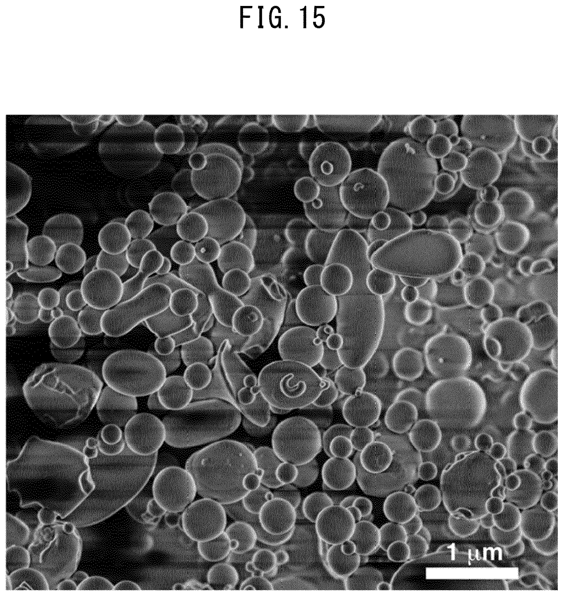

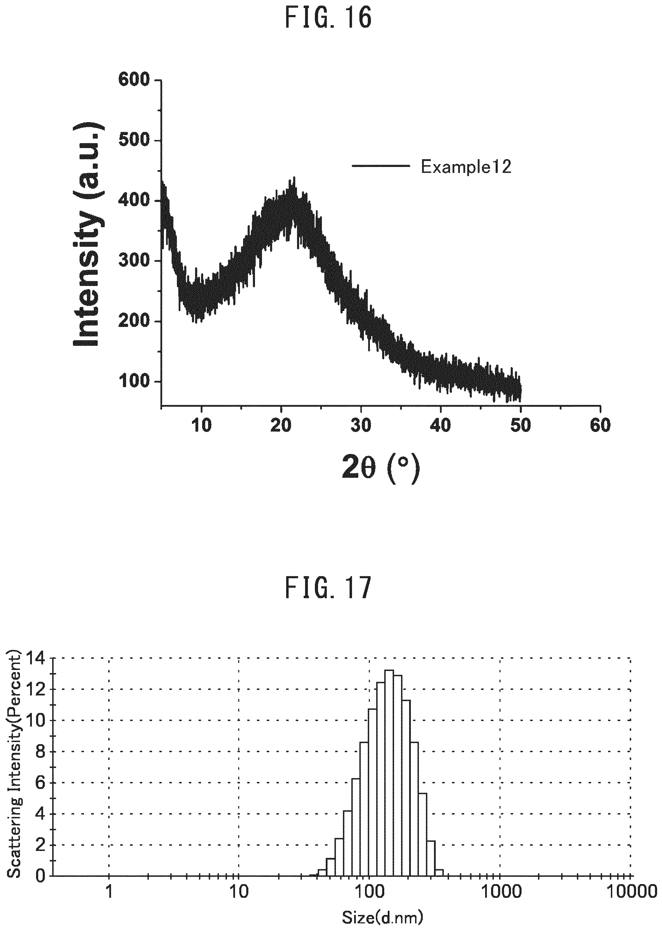

Formoterol fumarate hydrate was dissolved in THF-methanol (9:1 (v/v)) to prepare a 2.5 mM solution, and 10 mL of the solution was placed in a 50-mL glass bottle. While the solution in the glass bottle was vigorously stirred (400 rpm) by a magnetic stirrer, 40 mL of purified water was continuously injected at room temperature over 1 minute to be mixed. Stirring was stopped, and analysis by a DLS method was performed to provide particle size distribution (particle size: 145 nm, PDI: 0.052) in Figure 14. The resulting liquid was concentrated under reduced pressure by a rotary evaporator to provide a concentrated liquid, and the concentrated liquid was freeze-dried to provide amorphous particles as a white powder, comprising formoterol fumarate as a constituent component. An SEM sample was prepared on double-faced carbon tape, fixed to an aluminum pin stub and then observed by SEM to provide a spherical particle image illustrated in Figure 15. Furthermore, the powder was subjected to powder X-ray diffraction measurement, and was found to be amorphous by a diffraction pattern illustrated in Figure 16.

### [Example 13]

### Preparation of amorphous particles comprising formoterol fumarate as constituent component

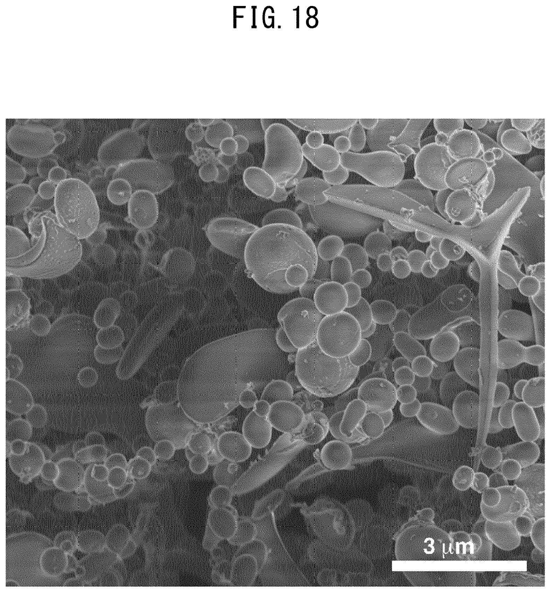

Formoterol fumarate was dissolved in methanol to prepare a 5 mM solution, and 1 mL of the solution was placed in a glass bottle having a volume of 5 mL. While the solution in the glass bottle was vigorously stirred (400 rpm) by a magnetic stirrer, 4 mL of purified water was continuously injected into the glass bottle at room temperature for 10 minutes. Stirring was stopped, and analysis by a DLS method was performed to provide particle size distribution (particle size: 120 nm, PDI: 0.183) in Figure 17. The resulting liquid was concentrated under reduced pressure by a rotary evaporator to provide a concentrated liquid, and the concentrated liquid was freeze-dried to provide amorphous particles as a white powder, comprising formoterol fumarate as a constituent component. An SEM sample was prepared on double-faced carbon tape, fixed to an aluminum pin stub and then observed by SEM to provide a spherical particle image illustrated in Figure 18. Furthermore, the powder was subjected to powder X-ray diffraction measurement, and was found to be amorphous by a diffraction pattern illustrated in Figure 19.

### [Comparative Example 1]

### Effect by modification of injection method of THF solution of organic compound into water

When the THF solution of PhH was dropped on a water surface in Example 1, a crystal particle of PhH was formed. Whether the particle was crystalline or not was determined by powder X-ray diffraction.

### Industrial Applicability

According to the present invention, submicrometer-sized spherical amorphous particles comprising a low molecular organic compound can be prepared simply with high reproducibility. The resulting particles are widely applicable in the pharmaceutical manufacturing field, printing field, organic electronic device field, etc.

### Explanation of References

- 1a:: liquid inlet channels
- 1b:: liquid inlet channels
- 2:: confluence passage
- 3:: microreactor

## Claims

1. Spherical water-dispersible amorphous particles having particle size of 10 nm to 990 nm and having a PDI of 0.01 to 0.5, comprising an organic compound having a molecular weight of 50 to 1500.

2. The amorphous particles according to claim 1, wherein the organic compound has a logP of 2.0 or more.

3. The amorphous particles according to claim 1 or 2, wherein the organic compound has a solubility in water of 0.3 mg/ mL or less at 25°C.

4. The amorphous particles according to any one of claims 1 to 3, wherein the organic compound is selected from the group consisting of formoterol, lansoprazole, cholesterol, simvastatin and telmisartan, and pharmaceutically acceptable salts thereof and solvates thereof.

5. The amorphous particles according to any one of claims 1 to 4, wherein the amorphous particles are a dried product.

6. A method for preparing the amorphous particles according to any one of claims 1 to 5, comprising the following steps of:
(1) dissolving an organic compound in a water-miscible organic solvent to prepare an organic solution; and
(2) introducing either the organic solution or water to the other, and mixing the organic solution and water to prepare a dispersion of the amorphous particle.

7. The method according to claim 6, wherein the concentration of the organic compound in the organic solution prepared in the step (1) is 0.1 µM to 1000 mM.

8. The method according to claim 6 or 7, wherein the mixing of the organic solution and water in the step (2) is performed by injecting the organic solution into water while stirring water.

9. The method according to claim 8, wherein the injection speed of the organic solution to X mL of water is 0.01X mL/min to 10X mL/min.

10. The method according to claim 6 or 7, wherein the mixing of the organic solution and water in the step (2) is performed by injecting water into the organic solution while stirring the organic solution.

11. The method according to claim 10, wherein the injection speed of water to Y mL of the organic solution is 0.01Y mL/min to 10Y mL/min.

12. The method according to claim 6 or 7, wherein the mixing of the organic solution and water in the step (2) is performed by contacting the organic solution and water at the interface between them while flowing them.

13. The method according to claim 12, wherein the mixing of the organic solution and water in the step (2) is performed by use of a microreactor.

14. The method according to any one of claims 6 to 13, further comprising stirring the dispersion of the amorphous particles prepared in the step (2) at 1 to 60°C to thereby increase the particle size of the amorphous particle.

15. The method according to any one of claims 6 to 14, further comprising evaporating the organic solvent in the dispersion of the amorphous particles prepared in the step (2) to prepare a water dispersion of the amorphous particle.

16. The method according to any one of claims 6 to 15, further comprising preparing a dried product of the amorphous particles from the dispersion of the amorphous particle.
